# EUROPÄISCHE PATENTSCHRIFT

(11) **EP 1 818 039 B1**
(45) Veröffentlichungstag und Bekanntmachung des Hinweises auf die Patenterteilung: **31.07.2013**
(21) Anmeldenummer: 07001577.1
(22) Anmeldetag: 25.01.2007
(51) Int. Cl.: A61J 3/10, A61K 9/00

(54) **Verfahren zur Herstellung eines lokalen Wirkstofffreisetzungssystems**
A method for the manufacture of a local drug-release system
Procédé de fabrication d'un système de libération de substance active local

(30) Priorität: 10.02.2006 DE 102006006510
(43) Veröffentlichungstag der Anmeldung: 15.08.2007
(73) Patentinhaber: Heraeus Kulzer GmbH, 63450 Hanau (DE)
(72) Erfinder: Kühn, Klaus-Dieter, Dr., 35041 Marburg (DE); Vogt, Sebastian, Dr., 99084 Erfurt (DE)
(74) Vertreter: Kühn, Hans-Christian

(56) Entgegenhaltungen:
- EP-A1- 0 061 108
- WO-A1-99/21595
- DE-A1- 2 320 373
- DE-A1- 2 651 441
- DE-A1- 19 641 775
- DE-B1- 2 917 037
- GB-A- 2 285 924
- US-A- 4 233 287

## Beschreibung

Der Gegenstand der Erfindung ist ein Verfahren zur Herstellung eines lokalen Wirkstofffreisetzungssystem, das aus annähernd kugelförmigen oder rotationssymmetrischen Körpern besteht, die im Wesentlichen aus Polymethylmethacrylat oder Polymethylmethacrylat-co-methylacrylat, Zirkoniumdioxid oder Bariumsulfat und einem oder mehreren pharmazeutischen Wirkstoffen aufgebaut sind.

Eine der schwierigsten Herausforderungen der Knochenchirurgie stellt auch gegenwärtig die Behandlung der Osteomyelitis dar. Osteomyelitis kann hämatogen, posttraumatisch oder postoperativ entstehen. Besonders schwierig ist die chronische Verlaufsform der Osteomyelitis zu behandeln, die im Extremfall zum Verlust von Gliedmaßen und bis zur Sepsis führen kann.

Üblich ist die die chirurgische Sanierung durch radikales Debridment. Dabei wird der infizierte beziehungsweise nekrotische Knochen weiträumig abgetragen. Im Folgenden wird die Knochenkavität mit einem lokalen Antibiotika-Träger ausgefüllt oder durch eine wiederholte Saug-Spül-Drainage behandelt. Durch lokale Freisetzung hoher Antibiotika-Mengen werden bei Verwendung eines hinreichend knochengängigen bakteriziden Antibiotikums, wie Gentamicinsulfat und Clindamycinhydrochlorid, auch die in den anliegenden Knochenarealen verblieben bakteriellen Keime wirksam bekämpft.

Kugelförmige lokale Wirkstofffreisetzungssysteme, die aus Polymethylmethacrylat, Zirkoniumdioxid und einem Antibiotikum aufgebaut sind, wurden erstmals 1975 von Klaus Klemm beschrieben (DE 23 20 373). Dieses Konzept erwies sich prinzipiell als erfolgreich, nachteilig war jedoch, dass nur ein geringer Teil des in den Kugeln enthaltenden Wirkstoffes freigesetzt wurde.

In Weiterentwicklung dieser Wirkstoffträger wurde 1978 von Heuser und Dingeldein vorgeschlagen, Glycin oder andere Aminosäuren zur Verbesserung der Antibiotikum-Freisetzung zuzusetzen (DE 26 51 441). Die inkorporierten Aminosäuren gehen nach Kontakt mit Wundsekret in Lösung und bilden Porensysteme, aus denen der Wirkstoff heraus diffundieren kann. Dadurch wurde eine verbesserte Wirkstofffreisetzung erreicht.

Lokale Wirkstofffreisetzungssysteme, die hauptsächlich aus Polymethylmethacrylat, einem Röntgenopaker und einem Antibiotikum aufgebaut sind, lassen sich entweder durch ein spezielles Spritzgießverfahren (DE 23 20 373) oder auch durch Gießen von Antibiotikum enthaltenden Polymethylmethacrylat-Knochenzementen in spezielle Formen herstellen (EP 796 712). Das Spritzgießen ist mit dem entscheidenden Nachteil verbunden, dass Temperaturen > 120 °C erforderlich sind, um das Polymer aufzuschmelzen. Dadurch ist es nicht möglich, thermisch labile Antibiotika oder andere thermische labile Wirkstoffe in diese lokalen Wirkstofffreisetzungssysteme zu integrieren. Dadurch ist bisher nur das mit Gentamicin beladene, durch konventionelles Spritzgießen hergestellte Wirkstofffreisetzungssystem unter dem Namen Septopal^{®} auf dem Markt. Gentamicin ist ein außerordentlich thermisch stabiles Antibiotikum. Im Hinblick auf die zunehmende Verbreitung von resistenten und insbesondere multiresistenten Bakterien sind jedoch weitere Antibiotika in lokalen Wirkstofffreisetzungssystemen wünschenswert. Leider sind diese Antibiotika, wie zum Beispiel Vancomycin und Teicoplanin, thermisch instabil. Dadurch ist es bisher nicht möglich, mit diesen Antibiotika lokale Wirkstofffreisetzungssysteme durch Spritzgießen herzustellen.

Eine Alternative dazu wird in EP 796 712 vorgeschlagen, wonach Implantatmaterialien unter Verwendung thermisch labiler Wirkstoffe hergestellt werden können. Dabei wird ein konventioneller PMMA-Knochenzement mit einem oder mehreren Antibiotika vermischt und in entsprechende Formen aus zum Beispiel Kunststoff überführt. Konventionelle PMMA-Knochenzemente bestehen aus einer Pulverkomponente, die aus einem Polymerpulver, einem Röntgenopaker und eine Polymerisationsinitiator zusammengesetzt ist, und einer flüssigen Monomerkomponente, die Methylmethacrylat, einen Stabilisator und einen Polymerisationsaktivator enthält. Nach Vermischen beider Komponenten treffen der Polymerisationsaktivator und der Polymerisationsinitiator aufeinander und die radikalische Polymerisation des Methylmethacrylates wird ausgelöst. Nach wenigen Minuten ist der PMMA-Knochenzement ausgehärtet. Durch dieses Aushärtungsverhalten können mit den in EP 796 712 vorgeschlagenen Formen unter Verwendung konventioneller PMMA-Knochenzemente kettenförmige Wirkstofffreisetzungssysteme nur in einem diskontinuierlichen Prozess hergestellt werden. Eine kontinuierliche Fertigung unter industriellen Bedingungen ist daher nicht möglich. Bei diesem Herstellungsverfahren wird N,N-Dimethyl-p-toluidin als Polymerisationsaktivator im PMMA-Knochenzement eingesetzt.

Der Erfindung liegt die Aufgabe zu Grunde, ein Verfahren zur Herstellung eines lokal wirksamen Wirkstofffreisetzungssystem zu entwickeln, das kontinuierlich durchgeführt werden kann. Das Herstellungsverfahren soll es ermöglichen, auch thermisch labile Antibiotika in die Wirkstofffreisetzungssysteme zu integrieren. Es sollen die Nachteile der in DE 23 20 373 und EP 796 712 beschriebenen Verfahren überwunden werden.

Die Aufgabe wurde in der Weise gelöst, dass Verfahren zur Herstellung eines lokalen Wirkstofffreisetzungssystem entwickelt wurde, das aus kugelförmigen Körpern besteht, die im Wesentlichen aus Polymethylmethacrylat oder Polymethylmethacrylat-co-methylacrylat, Zirkoniumdioxid oder Bariumsulfat und einem pharmazeutischen Wirkstoff aufgebaut sind, und die durch radikalische Polymerisation hergestellt sind, wobei im Temperaturbereich von 10-80 °C wirksame, radikalische Polymerisationsaktivatoren oder Reste solcher Polymerisationsaktivatoren, besonders aus den Gruppen der aromatischen Amine, der Schwermetallsalze und der Barbiturate, nicht enthalten sind, wobei durch Vermischen von Methylmethacrylat, Polymethylmethacrylat oder Polymethylmethacrylat-co-methylacrylat, Zirkoniumdioxid und/oder Bariumsulfat, einem oder mehreren pharmazeutischen Wirkstoffen und einem thermisch zerfallenden radikalischen Initiator eine Paste hergestellt wird, wobei a) die Paste eine solche Viskosität besitzt, dass diese bei Raumtemperatur durch Einwirkung der Schwerkraft nicht verformt werden kann, b) die annähernd kugelförmigen oder rotationssymmetrischen Körper auf einen Draht aufgespritzt werden, und die Körper auf eine Temperatur erwärmt werden, bei welcher der Polymerisationsinitiator zerfällt.

Insbesondere sind in dem erfindungsgemäßen Wirkstofffreisetzungssystem N,N-Dimethyl-anilin, N,N-Dimethyl-p-toluidin, N,N,-Bis-hydroxyethyl-p-toluidin oder deren beim Auslösen der radikalischen Polymerisation entstehenden Folgeprodukte nicht enthalten.

Die Erwärmung kann dabei z.B. durch Einwirkung von infraroter Strahlung oder durch Einwirkung von heißer Luft oder durch Einwirkung von Mikrowellen bewirkt werden.

Von Bedeutung ist es, wenn die mit der Paste hergestellten Körper vor der Aushärtung mechanisch so stabil sind, dass diese sich nicht auf Grund ihrer Eigenmasse durch Einwirkung der Schwerkraft deformieren, oder wenn die Körper auf Fäden gespritzt sind, sich von den Fäden ablösen.

Als thermisch zerfallende radikalische Initiatoren kommen dem Fachmann geläufige in Frage, besonders solche aus der Gruppe bestehend aus Dibenzoylperoxid, Dilauroylperoxid und Azoisobutyrodinitril.

Bei Schritt b) wird vorzugsweise ein Draht verwendet, der auf eine Temperatur im Bereich der Zerfallstemperatur des thermischen Initiators vorgewärmt ist. Durch das Vorwärmen des Drahtes kann erreicht werden, dass die Polymerisation im Inneren der gespritzten Körper gestartet wird, bevor die Aushärtung durch Einwirkung von infraroter Strahlung, heißer Luft oder durch Mikrowellen bewirkt wird. Dadurch haften die Körper besonders stabil auf dem Draht.

Das Spritzgießwerkzeug ist vorzugsweise aus Teflon oder einem anderen inerten Kunststoff gefertigt.

Die Erfindung wird durch nachstehende Beispiele erläutert, ohne jedoch die Erfindung zu beschränken.

### Beispiel 1

Es wird eine Paste aus 570,0 g Polymethylmethacrylat-co-methylacrylat (Molmasse ~ 800.000 g/mol), 285,0 g Methylmethacrylat, 89,0 g Zirkoniumdioxid, 42,0 g Gentamicinsulfat (AK 600), 8.8 g einer Mischung aus Dibenzoylperoxid und Wasser im Gewichtsverhältnis 3 :1 und 15,0 g Glycin durch intensives Vermischen hergestellt. Mit dieser viskosen Paste werden kontinuierlich mit Hilfe einer Spritzgießvorrichtung auf einen polyfilen, chirurgischen Stahldraht annähernd kugelförmige Körper mit einem Durchmesser von 7 mm gespritzt. Der Spritzgießprozess läuft bei Raumtemperatur ab. Danach werden die Körper in einem Trockentunnel bei einer Temperatur von 80 °C gehärtet. Die entstandenen Körper haben eine Masse von - 240 mg.

### Beispiel 2

Es wird eine Paste aus 570,0 g Polymethylmethacrylat-co-methylacrylat (Molmasse ~ 800.000 g/mol), 285,0 g Methylmethacrylat, 89,0 g Zirkoniumdioxid, 45 g Vancomycinhydrochlorid, 8.8 g einer Mischung aus Dibenzoylperoxid und Wasser im Gewichtsverhältnis 3 :1, und 15,0 g Glycin durch intensives Vermischen hergestellt. Mit der entstandenen Paste werden kontinuierlich mit Hilfe einer Spritzgießvorrichtung auf einen polyfilen, chirurgischen Stahldraht annähernd kugelförmige Körper mit einem Durchmesser von 7 mm gespritzt. Unmittelbar danach werden die Körper kontinuierlich mit einem Heizstrahler gehärtet, dabei werden die gespritzten Körper auf 60-70 °C durch die einwirkende IR-Strahlung erwärmt und die Polymerisation wird ausgelöst. Die ausgehärteten Körper haben eine Masse von ~ 240 mg.

## Patentansprüche

1. Verfahren zur Herstellung eines lokalen Wirkstoffsystems, das aus annähernd kugelförmigen oder rotationssymmetrischen Körpern besteht, die im Wesentlichen aus Polymethylmethacrylat oder Polymethylmethacrylat-co-methylacrylat, Zirkoniumdioxid oder Bariumsulfat und einem oder mehreren pharmazeutischen Wirkstoffen, insbesondere Antibiotika, aufgebaut sind, und die durch radikalische Polymerisation hergestellt sind, wobei im Temperaturbereich von 10-80 °C wirksame, radikalische Polymerisationsaktivatoren oder Reste dieser Polymerisationsaktivatoren aus den Gruppen der aromatischen Amine, der Schwermetallsalze und der Barbiturate nicht enthalten sind, **dadurch gekennzeichnet, dass**
a) durch Vermischen von Methylmethacrylat, Polymethylmethacrylat oder Polymethylmethacrylat-co-methylacrylat, Zirkoniumdioxid und/oder Bariumsulfat, einem oder mehreren pharmazeutischen Wirkstoffen und einem thermisch zerfallenden radikalischen Initiator eine Paste hergestellt wird, wobei die Paste eine solche Viskosität besitzt, dass diese bei Raumtemperatur durch Einwirkung der Schwerkraft nicht verformt werden kann,
b) die annähernd kugelförmigen oder rotationssymmetrischen Körper auf einen Draht aufgespritzt werden, und
c) die Körper auf eine Temperatur erwärmt werden, bei welcher der Polymerisationsinitiator zerfällt.

2. Verfahren nach Anspruch 1, wobei in Schritt c) die Erwärmung durch Einwirkung von infraroter Strahlung oder durch Einwirkung von Luft oder durch Einwirkung von Mikrowellen bewirkt wird.

3. Verfahren nach Anspruch 1 oder 2, **dadurch gekennzeichnet, dass** ein oder mehrere Stoffe der Gruppe Dibenzoylperoxid, Dilauroylperoxid und Azoisobutyrodinitril als thermisch zerfallender radikalischer Initiator verwendet werden.

4. Verfahren nach mindestens einem der Ansprüche 1 bis 3, **dadurch gekennzeichnet, dass** beim Schritt b) ein Draht verwendet wird, der auf eine Temperatur im Bereich der Zerfallstemperatur des thermischen Initiators vorgewärmt ist.

5. Verfahren nach mindestens einem der Ansprüche 1 bis 4, **dadurch gekennzeichnet, dass** das Spritzgießwerkzeug aus Teflon oder einem anderen inerten Kunststoff gefertigt ist.

6. Verfahren nach einem der Ansprüche 1 bis 5, **dadurch gekennzeichnet, dass** in dem lokalen Wirkstofffreisetzungssystem N,N-Dimethyl-anilin, N,N-Dimethyl-p-toluidin, N,N-Bis-hydroxyethyl-p-Toluidin oder deren beim Auslösen der radikalischen Polymerisation entstehenden Folgeprodukte nicht enthalten sind.

## Claims

1. Method for producing a local active substance system that consists of approximately spherical or rotationally-symmetrical bodies that are essentially made up of polymethylmethacrylate or polymethylmethacrylate-co-methylacrylate, zirconium dioxide or barium sulfate, and one or more pharmaceutically active substances, in particular antibiotics, and are produced by means of radical polymerisation, whereby no radical polymerisation activators or residues of said polymerisation activators that are effective in the temperature range of 10-80 °C and are members of the groups of aromatic amines, heavy metal salts, and barbiturates are contained therein, **characterised in that**
a) a paste is produced through mixing methylmethacrylate, polymethylmethacrylate or polymethylmethacrylate-co-methylacrylate, zirconium dioxide and/or barium sulfate, one or more pharmaceutically active substances, and a thermally disintegrating radical initiator, whereby the viscosity of the paste is appropriate such that the paste cannot be deformed at room temperature through the action of gravity;
b) the approximately spherical or rotationally-symmetrical bodies are injection-moulded onto a wire; and
c) the bodies are heated to a temperature at which the polymerisation initiator disintegrates.

2. Method according to claim 1, whereby the heating in step c) is effected through the action of infrared radiation or through the action of air or through the action of micro-waves.

3. Method according to claim 1 or 2, **characterised in that** one or more substances from the group of dibenzoylperoxide, dilauroylperoxide, and azoisobutyrodinitrile are used as thermally disintegrating radical initiator.

4. Method according to at least one of the claims 1 to 3, **characterised in that** a wire pre-heated to a temperature in the range of the disintegration temperature of the thermal initiator is used in step d).

5. Method according to at least one of the claims 1 to 4, **characterised in that** the injection moulding tool is manufactured from Teflon or another inert plastic material.

6. Method according to any one of the claims 1 to 5, **characterised in that** the local active substance release system does not contain N,N-dimethylaniline, N,N-dimethyl-p-toluidine, N,N-bis-hydroxyethyl-p-toluidine or the products derived from these substances during the initiation of the radical polymerisation.

## Revendications

1. Procédé de fabrication d'un système de substances actives local qui se compose de corps approximativement sphériques ou à symétrie de rotation qui sont construits essentiellement à partir de méthacrylate de polyméthyle ou de polyméthylméthacrylate-co-méthylacrylate, dioxyde de zirconium ou sulfate de baryum et d'une ou plusieurs substances actives pharmaceutiques, notamment antibiotiques, et qui sont fabriqués par polymérisation radicalaire, dans lequel des activateurs de polymérisation radicalaire efficaces dans la plage de température de 10 à 80°C ou des résidus de ces activateurs de polymérisation provenant des groupes des amines aromatiques, des sels de métaux lourds et des barbituriques ne sont pas contenus, **caractérisé en ce que**
a) une pâte est fabriquée par mélange de méthacrylate de méthyle, méthacrylate de polyméthyle ou polyméthylméthacrylate-co-méthylacrylate, dioxyde de zirconium et/ou sulfate de baryum, d'une ou plusieurs substances actives pharmaceutiques et d'un initiateur radicalaire à décomposition thermique, dans lequel la pâte possède une viscosité telle que celle-ci ne peut pas être déformée à température ambiante par action de la force de gravité,
b) les corps approximativement sphériques ou à symétrie de rotation sont pulvérisés sur un fil métallique et
c) les corps sont chauffés à une température à laquelle l'initiateur de polymérisation se décompose.

2. Procédé selon la revendication 1, dans lequel, à l'étape c), le chauffage est effectué par action de rayonnement infrarouge ou par action d'air ou par action de micro-ondes.

3. Procédé selon la revendication 1 ou 2, **caractérisé en ce qu'**une ou plusieurs substances du groupe du peroxyde de dibenzoyle, peroxyde de dilauroyle et azoisobutyrodinitrile sont utilisées en tant qu'initiateur radicalaire à décomposition thermique.

4. Procédé selon au moins une des revendications 1 à 3, **caractérisé en ce que**, à l'étape b), un fil métallique qui est préchauffé à une température dans la plage de la température de décomposition de l'initiateur thermique est utilisé.

5. Procédé selon au moins une des revendications 1 à 4, **caractérisé en ce que** l'outil de moulage par injection est réalisé en Téflon ou un autre plastique inerte.

6. Procédé selon l'une quelconque des revendications 1 à 5, **caractérisé en ce que** de la N,N-diméthyl-aniline, N,N-diméthyl-p-toluidine, N,N-bis-hydroxyéthyl-p-toluidine ou leurs produits secondaires qui surviennent lors du déclenchement de la polymérisation radicalaire ne sont pas contenus dans le système de libération de substances actives local.
